# EUROPEAN PATENT APPLICATION

(11) **EP 4 837 512 A1**
(43) Date of publication of application: **11.11.2026**
(21) Application number: 25175001.4
(22) Date of filing: 08.05.2025
(51) Int. Cl.: A61B 8/00, G16H 50/20

(54) **ULTRASOUND SYSTEM HAVING A TRAINED ARTIFICIAL INTELLIGENCE (AI) MODEL AND METHOD OF USE**

(71) Applicant: Koninklijke Philips N.V., 5656 AE Eindhoven (NL)
(72) Inventor: XIE, Hua, Eindhoven (NL); ERRICO, Claudia, Eindhoven (NL); MENG, Yishuang, 5656AG Eindhoven (NL); BAI, Xianghui, Eindhoven (NL); SHIH, Cho-Chiang, Eindhoven (NL); SHETTIGAR, Srikanth, Eindhoven (NL); BRUNELLE, Elizabeth, Eindhoven (NL)
(74) Representative: Philips Intellectual Property & Standards

(57) **Abstract**

An ultrasound system and method include a trained AI model to provide a measure of quality assurance of a measurement using an ultrasound system is described. When a measurement difference is greater than the threshold, or leads to a clinically significant difference, the system and method provide an alert indicative of the difference between the trained AI model measurement and a correction, or provide an alert indicative of the clinically significant difference. The US system and method archives paired images comprising the trained AI model measurement and the correction to the trained AI model measurement. The US system and method determines a measure of quality assurance based on the difference between the trained AI model measurement and the correction to the trained AI model measurement.

## Description

### FIELD OF THE INVENTION

This disclosure relates to an ultrasound system comprising a trained artificial intelligence (AI) model adapted to provide a measure of quality assurance of a measurement and user training, and a method of use.

### BACKGROUND OF THE INVENTION

Artificial intelligence (AI) technology has been progressively integrated into ultrasound imaging. AI technology has been progressively integrated into ultrasound systems to automate workflow for improved user productivity and inter-operator acquisition consistency. One main application is to replace tedious manual measurement of organ / anatomy dimensions with AI technology (e.g. Machine Learning / Deep Learning) enabled auto-measure tools. Representative applications that can benefit from AI measurements include but are not limited to abdominal organ (e.g., length, width, height, and volume based on these measurements) measurement in radiology (e.g. renal length, liver length, gallbladder wall thickness); fetal biometric parameters in obstetrics (e.g. abdominal circumference, head circumference, femur length); and 2D echocardiographic measurement for cardiology (e.g. left ventricular internal end diastolic dimension, left ventricular internal end systolic dimension) to name only a few.

As a built-in safety mechanism, many AI features offer users the freedom to review AI output and make necessary changes. Moreover, the measurements made by different users can vary greatly depending on the experience, and even current state of fatigue of the users to perform the desired measurement. Accordingly, AI measurements based on models trained using a significant amount of ground truth data (GTD) from many scans/measurements of the organ being scanned. Using the trained AI model, calipers positions are predicted and automatically displayed. At this point, the user can either fully accept the auto-measure results from the AI, or adjust caliper locations directly on a user interface when he/she is not satisfied with the locations generated by the AI algorithm. In both cases, the user then proceeds to lock the AI generated calipers, acquire the image with the calipers and corresponding measurement, and send the results to the report, or lock the user adjusted calipers, acquire the image with the calipers and corresponding measurement, and send the results to the report. At the last step, ultrasound images and a measurement report may be sent to radiologist, or other clinician for review and interpretation.

Unfortunately, known AI models may be unreliable. Performance issues may arise during scan sessions including but not limited to as poor predictions by the AI model on certain subjects / cases and challenges handling data from more recent scans.

What is needed, therefore, is a method of training an AI model that improves the accuracy of the measurements made therewith to an acceptable performance level.

### SUMMARY OF THE INVENTION

The invention is defined by the independent claims. Advantageous embodiments are provided in the dependent claims.

According to a representative embodiment, a method of using a trained AI model to provide a measure of quality assurance of a measurement using an ultrasound image is described. The method comprises: applying the trained AI model to an ultrasound image to acquire automatically a trained AI model measurement of an anatomy. The automatically acquired trained AI model measurement of the anatomy provides a pair of calipers positioned on the ultrasound image to obtain a length or distance measurement. The method further comprises: detecting when a user corrects the trained AI model measurement by adjusting a caliper position to obtain a correction to the trained AI model measurement; determining a measurement difference between the trained AI model measurement and the correction to the trained AI model measurement; and determining when a measurement difference (or discrepancy) between the trained AI model measurement and the correction to the trained AI model measurement is greater than a threshold. When the measurement difference between the trained AI model measurement and the correction is greater than the threshold, or leads to a different clinical decision, the method further comprises: providing an alert indicative of the measurement difference between the trained AI model measurement and the correction, or providing an alert indicative of the different clinical decision; archiving paired images comprising the trained AI model measurement and the correction to the trained AI model measurement; and determining an improvement to the trained AI model based on the measurement difference between the trained AI model measurement and the correction to the trained AI model measurement. The above method may, in particular, be computer-implemented. The method of the present invention provides a measure of quality assurance by automatically acquiring measurements and detecting discrepancies between AI-generated measurements and user corrections, which ensures that the measurements are accurate and reliable.

In some embodiments, the method further comprises: determining when the trained AI model measurement and the correction to the trained AI model measurement lead to a clinically significant difference, by evaluating the measurement with reference to a normal range. In this way, more informed clinical decisions are advantageously based on accurate measurements.

In some embodiments, normal ranges are provided for a selected measurement defined by clinical guidelines or by an institutional guideline. In such embodiments, the method further comprises: comparing the normal ranges to the trained AI model measurement, and/or the correction to the trained AI model measurement. This ensures that the measurements are within acceptable limits and may help in identifying abnormalities.

In some embodiments, the measurement difference comprises a difference between the length or distance measurement and a maximum caliper position difference.

In some embodiments, the method further comprises: providing a set of thresholds related to measurement variability. In such embodiments, thresholds in the set of thresholds may preferably be pre-defined specifically for an individual organ, an image view, anatomical measurement parameters, and caliper positions. In this way better consistency and accuracy in the measurements can be ensured.

In some embodiments, the method further comprises: determining, with the trained AI model, a performance evaluation of a user based on the difference between the length or distance measurement and the maximum caliper position difference. In such embodiments, determining the performance evaluation of the user may be further based on the difference between the caliper position and the correction to the position. In this manner areas where the user may need further training or improvement may be advantageously identified.
In some embodiments, the method further comprises: retraining the trained AI model with archived data from a plurality of measurements. This makes it possible to continuously update and improve the AI model based on new data.

In some embodiments, the method further comprises: determining a difference between the caliper position and a correction to the position; determining when the caliper position and the correction is greater than a threshold; and when the difference is greater than the threshold providing an alert indicative of the difference exceeding the threshold. In such embodiments, the method may preferably further comprise: determining a measure of quality assurance based on the difference between the caliper position and the correction to the position. A measure of quality assurance is useful for maintaining high standards of measurement accuracy.

In some embodiments, the method further comprises: providing a user prompt to enter reasons for the correction; receiving the reasons; and archiving the reasons with the archived paired images. This is advantageous to better understand the context for the corrections and improving the AI model.

In such embodiments, the method may further comprise: transmitting to a remote system the ultrasound image with the trained AI model measurement of the anatomy, the correction to the trained AI model measurement and the receive reason, to perform re-training of the trained AI model, or output a measurement quality report at the remote system, or both. By transmitting the ultrasound image with the trained AI model measurement, the correction, and the reason for the correction to a remote system for retraining the AI model or outputting a measurement quality report, continuous improvement and quality management can be advantageously achieved.

According to another representative embodiment, a tangible, non-transitory computer readable medium is described. The tangible, non-transitory computer readable medium stores instructions, which when executed by a processor, cause the processor to perform any of the above-described methods.

According to yet another representative embodiment, an ultrasound system comprising a trained AI model to provide a measure of quality assurance of a measurement using an ultrasound system is described. The ultrasound system comprises: a processor; a tangible, non-transitory computer readable medium that stores instructions, which when executed by the processor, cause the processor to: apply the trained AI model to an ultrasound image to acquire automatically trained AI model measurement of an anatomy, wherein the automatically acquired trained AI model measurement of the anatomy provides a pair of calipers positioned on the ultrasound image to obtain a length or distance measurement; detect when a user corrects the trained AI model measurement by adjusting a caliper position to obtain a correction to the trained AI model measurement; determine a measurement difference between the trained AI model measurement and the correction to the trained AI model measurement; determine when the measurement difference between the trained AI model measurement and the correction to the trained AI model measurement is greater than a threshold; when the measurement difference between the trained AI model measurement and the correction is greater than the threshold, or leads to a different clinical decision (such as a clinically significant difference), provide an alert indicative of the measurement difference between the trained AI model measurement and the correction, or provide an alert indicative of the different clinical decision; archive paired images comprising the trained AI model measurement and the correction to the trained AI model measurement; and determine an improvement to the trained AI model based on the measurement difference between the trained AI model measurement and the correction to the trained AI model measurement.

In some embodiments, the measurement difference comprises a difference between the length or distance measurement and a maximum caliper position difference.

In some embodiments, a set of thresholds related to measurement variability is provided. In such embodiments, thresholds in the set of thresholds may preferably be pre-defined specifically for individual organ, image view, anatomical measurement parameters, and caliper positions.

In some embodiments, normal ranges are provided for a selected measurement defined by clinical guidelines or by an institutional guideline, and the instructions further cause the processor to compare the normal ranges to the trained AI model measurement, and/or the corrected trained AI model measurement.

In some embodiments, the instructions further cause the processor to determine, with the trained AI model, a performance evaluation of a user based on the difference between the length or distance measurement and the maximum caliper position difference. In such embodiments, the performance evaluation of the user may preferably be further based on the difference between the caliper position and the correction to the position.

In some embodiments, the instructions further cause the processor to retrain the trained AI model with archived data from a plurality of measurements.

In some embodiments, the instructions further cause the processor to: calculate determine a difference between the caliper position and a correction to the position; determine when the caliper position and the correction is greater than a threshold; and when the difference is greater than the threshold providing an alert indicative of the difference exceeding the threshold.

In some embodiments, the instructions further cause the processor to provide a user prompt to enter reasons for the correction; receive the reasons; and archive the reasons with the archived paired images.

According to yet another representative embodiment, an ultrasound imaging system comprises: an ultrasound imaging device; a processor; a display configured to display an ultrasound image with a measurement, and a user interface configured to receive an input of a user-provided measurement; and a tangible, non-transitory computer readable medium that stores a trained anatomical measurement model comprising instructions in the non-transitory computer readable medium, wherein when executed by the processor, the instructions cause the processor to: automatically obtain a measurement of anatomy in an ultrasound image; execute the instructions on the ultrasound image to automatically acquire the measurement of anatomy; obtain a user-provided measurement; determine a positional difference between the measurement of anatomy and the user-provided measurement; determine when the positional difference between the measurement of anatomy and the user-provided measurement is greater than a pre-determined measurement variability threshold or leads to a clinically significant difference; based on the determination, issue a user prompt at the ultrasound imaging system to provide a reason for a correction; and archive paired images comprising the measurement of the anatomy and the user-provided measurement; transmit to a remote system the ultrasound image with the measurement of the anatomy, the user-provided measurement, and the provided reason to perform re-training the trained anatomical measurement model, or output a measurement quality report at the remote system, or both.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a simplified block diagram of an ultrasound (US) system according to a representative embodiment.
Fig. 2 is a flow chart of a method of executing an AI model to provide a measure of quality assurance of a measurement in accordance with a representative embodiment.
Fig. 3A is a table showing examples of reference thresholds for liver size measurement specific to view and measurement parameters.
Fig. 3B is a table showing examples of reference thresholds for kidney size measurement specific to view and measurement parameters.
Fig. 3C is a table showing one example of spleen length reference range for normal, borderline splenomegaly and definite splenomegaly for adult subjects.
Fig. 4A is a US image auto-measure results by AI without any manual adjustment of a kidney length in longitudinal view.
Fig. 4B is a US image auto-measure results by AI without any manual adjustment of a spleen length in longitudinal view.
Fig. 5A is a US image showing user-adjusted caliper positions and new measurement different from AI prediction of a right liver AP thickness measurement with no significant difference between AI prediction and user measurement in accordance with a representative embodiment.
Fig. 5B is a US image showing user-adjusted caliper positions and new measurement significantly different from AI prediction of a kidney length measurement in a longitudinal view in accordance with a representative embodiment.
Fig. 6A is a US image showing a right kidney length measurement made by an AI model in longitudinal view in accordance with a representative embodiment.
Fig. 6B shows the US image of Fig. 6A including a user-corrected length measurement of Fig. 6A done by adjusting the position of both calipers in accordance with a representative embodiment.
Fig. 7 is a flow chart of a method of updating and retraining an AI computational model in accordance with a representative embodiment.

### DETAILED DESCRIPTION OF EMBODIMENTS

Aspects of the disclosure may be supported by various information technology (IT) backends, including either or both local architectures, either as monoliths, networked, or a combination thereof, and hosted architectures, such as a software as a service (SaaS), platform as a service (PaaS), and/or infrastructure as a service (IaaS), or the like. In an example, a supporting infrastructure includes multiple interconnected layers respectively hosting, as an abstraction, various IT processes, services, accounts, and other management components.

Any of the steps described in relation to examples and/or training described below can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. As an example, a computer system performs logical processing based on digital signals received via an analogue-to-digital converter.

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the various embodiments described herein provide an artificial intelligence (AI) based method and system adapted to continuously monitor and update AI enabled ultrasound auto-measure features post-deployment, such as after the US system has been placed into use for conducting ultrasound examinations in a clinical setting. As described more fully herein, the present teachings leverage user analytics and clinical guidelines for smart documentation, and archive ultrasound measurements when the operator adjusts AI measured outputs including changes in caliper position and measurements made during a live scan. User analytics including inter-user measurement variability are derived during AI model development and retraining, and validation stages specific to organs, parameters, and acquisition views. The user analytics may be combined with clinical guidelines as criteria to evaluate the discrepancy between AI results and user adjustments to the AI results, and raise an alert accordingly.

Among other improvements to the function of an ultrasound system and computer, and other technical fields, the present teachings enable systematical evaluation of the differences between AI caliper locations, and measurements and adjustments to the AI caliper locations and measurements made by the sonographer/clinician at the location of the US system. Moreover, the present teachings enable continuous monitoring of the AI model for the of the US system manufacturer that implements the AI feature. Specifically, and as described more fully below, the present teachings provide not only the ability to retrain completely an AI model based on a comparatively large amount of data from US scans of a plurality of patients and a plurality of sonographers/clinicians performing the US scans, but also contemplates tuning the AI model by adjusting hyperparameters used in the model to improve the performance of the AI model continually.

Furthermore, and as described more fully below, the methods and systems of the present teachings can be used to provide quality control of the US system, and to monitor the performance of individual sonographers/clinicians that perform the US scans. These reviews can also be used to aid in the training of sonographers and clinicians. Ultimately, the methods and systems of the present teachings improve the field of diagnostic imaging by enabling training and retraining of the sonographers and clinicians to improve the accuracy of caliper positions and measurements, and providing quality management for the ultrasound department in the healthcare institution.

Among other aspects, and as described more fully below, the present teachings include collecting from clinical studies and inter-user or inter-AI-user measurement variability collected from clinical studies and literature during AI model development and validation stages. When normal reference ranges for the selected measurement parameters are defined in clinical guidelines or by the healthcare institute, these are referenced for training and updating the AI model.

As described more fully herein, AI model improvement through retraining and updating by the US system manufacturer, and quality management by the healthcare institute, archives and algorithms may be stored on-cart or in the cloud. Notably, as used herein, "retraining" refers to the process of training a AI model with new datasets, and "updating" refers to the outcome of a new AI model with different weights and hyperparameters. Beneficially, one improvement to the US system after deploying the retrained AI model can be measured by the percentage of user corrections or the success rate of AI enabled Auto-Measure feature of the US system. If user accepts an AI output without the need to make adjustments during a procedure, then the AI success rate is 100%, where the success rate can be defined =100% *(1- number of user correction / the total number of AI measurement). While a 100% success is optimal, the continual retraining and updating of the trained AI model in accordance with various representative embodiments described below, provides a continual measure of the accuracy of the trained AI model. Furthermore, the ultrasound department in the healthcare institution can use these measures to monitor sonographer behavior and performance. For example, with certain review processes in place, a measure of which sonographers "correct" AI output more often than the rest can be made. If the correction is valid, ultrasound department can report those findings to the US system manufacturer to support model retraining. By contrast, if the correction in not valid, a potential indicator of a sonographer lacking experience may be realized, and further training may be provided to the sonographer.

Moreover, and as described more fully below, algorithms may be used to determine whether an Al-measurement or caliper position differs significantly after user adjustment from the AI measurement or caliper adjustment, or leads to a clinically significant difference. Clearly, the AI model execution, training, retraining and updating cannot be done practically in the human mind. Specifically, and as noted above, due at least to the complexity of the AI models and the comparatively large amount of data used, these computations could not be done by the human mind with sufficient accuracy and/or cannot be done practically in the human mind (e.g., within a certain period of time). Even if these computations could be done by hand in a matter of months or years, the delivery of the diagnosis would be too late to have meaningful use in the diagnosis and treatment of various medical conditions and diseases that are diagnosed in accordance with the US systems and methods described in connection with the various representative embodiments of the present teachings.

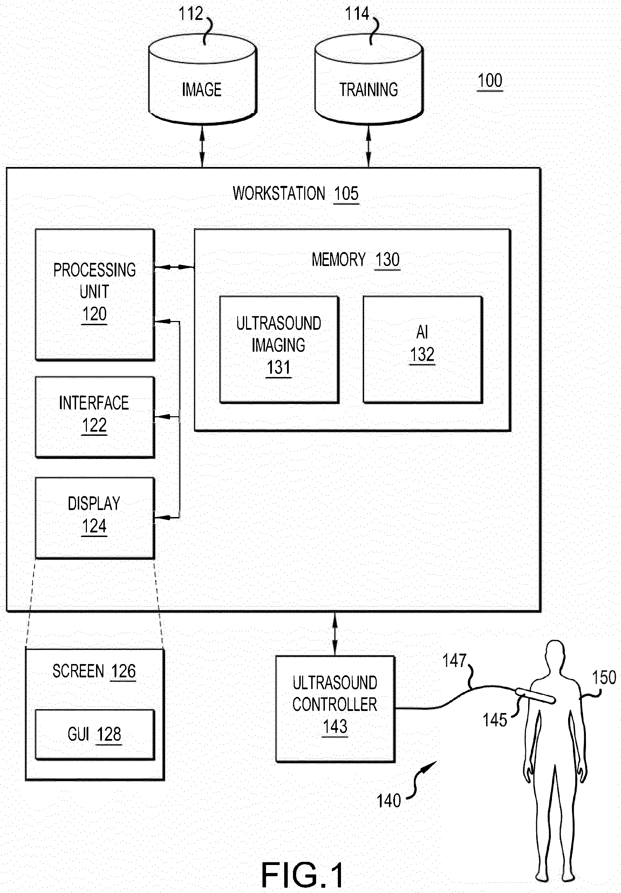

Fig. 1 is a simplified block diagram of an ultrasound (US) system according to a representative embodiment.

Referring to Fig. 1, US system 100 includes a workstation 105 for implementing and/or managing the processes described herein with regard to executing and training of AI models.

The workstation 105 includes one or more processors indicated by processing unit 120, one or more memories indicated by memory 130, a user interface 122 and a display 124. The processing unit 120 communicates with representative ultrasound imaging system 140 through an imaging interface (not shown). The ultrasound imaging system 140 includes an ultrasound controller 143 and an ultrasound probe 145 operable by an operator to obtain ultrasound images of various anatomies of a subject (patient) 150. The ultrasound probe 145 may be manipulated manually by the ultrasound user, automatically by a robot under control of a robot controller (not shown), or a combination of both.

The ultrasound probe 145 may include a 1D/2D matrix array of transducer elements, capable of scanning in two or three dimensions, for example, for emitting ultrasound waves into the body of the subject 150 and receiving echo signals in response. The transducer elements may include capacitive micromachined ultrasonic transducers (CMUTs) or piezoelectric transducers formed of materials such as lead zirconate titanate (PZT) or polyvinylidene difluoride (PVDF), for example, although other types of transducer material may be incorporated without departing from the scope of the present teachings. The transducer array may be coupled to a microbeamformer in the ultrasound probe 145, which controls transmission and reception of signals by the transducer elements.

The ultrasound probe 145 is connected to the ultrasound controller 143 via a probe cable 147. The ultrasound controller 143 is configured to control the ultrasound imaging process, and includes known elements for performing ultrasound imaging, such as a transmit/receive (T/R) switch configured to switch between transmission and reception modes, and a main beamformer configured to provide final beamforming. One of the functions performed by the ultrasound controller 143 is the direction in which beams are steered and focused. Generally, the transmitting of ultrasound signals and the receiving and processing of echo signals in response are known, and therefore additional detail in this regard is not included herein. In various embodiments, all or part of the functionality of the ultrasound controller 143 may be implemented by the processing unit 120.

The memory 130 stores instructions executable by the processing unit 120. When executed, the instructions cause the processing unit 120 to implement one or more processes for executing, continual retraining/updating of an AI model, user training and/or their quality assurance based on ultrasound images acquired by the ultrasound imaging system 140, including an automatically acquired trained AI model measurement of anatomy described more fully below. The ultrasound images may be provided from the ultrasound imaging system 140 in real-time or near real-time during a scanning procedure, or may be retrieved from storage following the scanning procedure. For purposes of illustration, the memory 130 is shown to include software modules, each of which includes the instructions, executable by the processing unit 120, corresponding to an associated capability of the US system 100, as discussed below.

The processing unit 120 is representative of one or more processing devices, and may be implemented by a general purpose computer, a central processing unit (CPU), a digital signal processor (DSP), a graphical processing unit, a computer processor, a microprocessor, a state machine, programmable logic device, field programmable gate arrays (FPGAs), application specific integrated circuits (ASICs), or combinations thereof, using any combination of hardware, software, firmware, hardwired logic circuits, or combinations thereof. Any processor or processing unit herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 130 may include main memory and/or static memory, where such memories may communicate with each other and the processing unit 120 via one or more buses. The memory 130 may be implemented by any number, type and combination of random access memory (RAM) and read-only memory (ROM), for example, and may store various types of information, such as software algorithms, AI machine learning models, and computer programs, all of which are executable by the processing unit 120. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

The US system 100 may also include databases for storing information that may be used by the various software modules of the memory 130. Representative databases may include an image database 112 for storing images from current and previous examinations of subjects and a training database 114 for storing training images for training the generative AI framework, as discussed more fully below. For example, the image database 112 may include image data from previously obtained ultrasound images of various subjects. Each of the image database 112 and the training database 114 may be implemented by any number, type and combination of RAM and ROM, for example, as discussed above with regard to the memory 130. The databases comprise tangible storage mediums for storing data and executable software instructions and is non-transitory during the time data and software instructions are stored therein. The databases may be secure and/or encrypted, or unsecure and/or unencrypted. For purposes of illustration, the image database 112 and the training database 114 are shown as separate storage media, although it is understood that all or some of the databases may be combined with one another and/or included in the memory 130, without departing from the scope of the present teachings.

The processing unit 120 may include or have access to an AI engine, which may be implemented as software that provides artificial intelligence (e.g., deep learning, neutral network models) and applies machine learning described herein. The AI engine may reside in any of various components in addition to or other than the processing unit 120, such as the memory 130, an external server, and/or the cloud, for example. When the AI engine is implemented in a cloud, such as at a data center, for example, the AI engine may be connected to the processing unit 120 via the internet or other communication network using one or more wired and/or wireless connection(s). In various embodiments, all or part of the processes provided by machine learning models and algorithms discussed herein may be implemented by the AI engine, for example. To this end, and as noted above, the training and execution of the machine learning models and algorithms cannot practically be performed in the human mind due at least to the complexity of the models and/or the large amount of digital image-related data required to train the models.

The user interface 122 is configured to provide information and data output by the processing unit 120, the memory 130 and/or the ultrasound imaging system 140 to the user and/or for receiving information and data input by the user. That is, the user interface 122 enables the user to enter data and to control or manipulate aspects of the processes described herein, and also enables the processing unit 120 to indicate the effects of the user's input, which may include control or manipulation of the ultrasound probe 145 by the user. All or a portion of the user interface 122 may be implemented by a graphical user interface (GUI), such as GUI 128 viewable on the display 124. The user interface 122 may include one or more interface devices, such as a mouse, a keyboard, a trackball, a joystick, a microphone, a video camera, a touchpad, a touchscreen, voice or gesture recognition captured by a microphone or video camera, for example. Of note, the user interface allows the user (sonographer or other clinician) to select certain images for not only the particular examination, but also for archiving and later use in retraining or updating the model and user training and user performance evaluation.

The display 124 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 124 includes a screen 126 for viewing ultrasound images of the subject 150, along with various features described herein to communicate to the user the degree of image degradation, if any, as well as the GUI 128 to enable the user to interact with the displayed images and features. In an embodiment, the ultrasound imaging system 140 may include a separate dedicated display for acquiring the ultrasound images, where dedicated display is also represented by the display 124.

Referring to the memory 130, the various modules store sets of data and instructions executable by the processing unit 120 to automatically acquire a trained AI model measurement of anatomy, and to execute the trained predictive algorithm as discussed more fully below. In the depicted illustrative embodiment, the memory includes ultrasound imaging module 131, which is configured to receive and process ultrasound images acquired by the ultrasound probe 145 of the ultrasound imaging system 140 to provide corresponding ultrasound image data. The ultrasound images are obtained by the user during corresponding ultrasound examinations, e.g., of the subject 150. The ultrasound images may be received in real-time or near real-time from the ultrasound imaging system 140, e.g., during a contemporaneous imaging session of the subject 150. The ultrasound images are displayed on the display 124, enabling the user to visualize the anatomy of the subject 150 while performing sweeps using the ultrasound probe 145. Alternatively, or additionally, the ultrasound images may be previously acquired images, obtained during a previous imaging session of the subject 150 and retrieved from storage (e.g., image database 112). The ultrasound imaging module 131 may also store data associated with the ultrasound images, such as time and date of image acquisition, identification of the ultrasound imaging system 140, and identification of the operator who acquired the ultrasound images using the ultrasound imaging system 140.

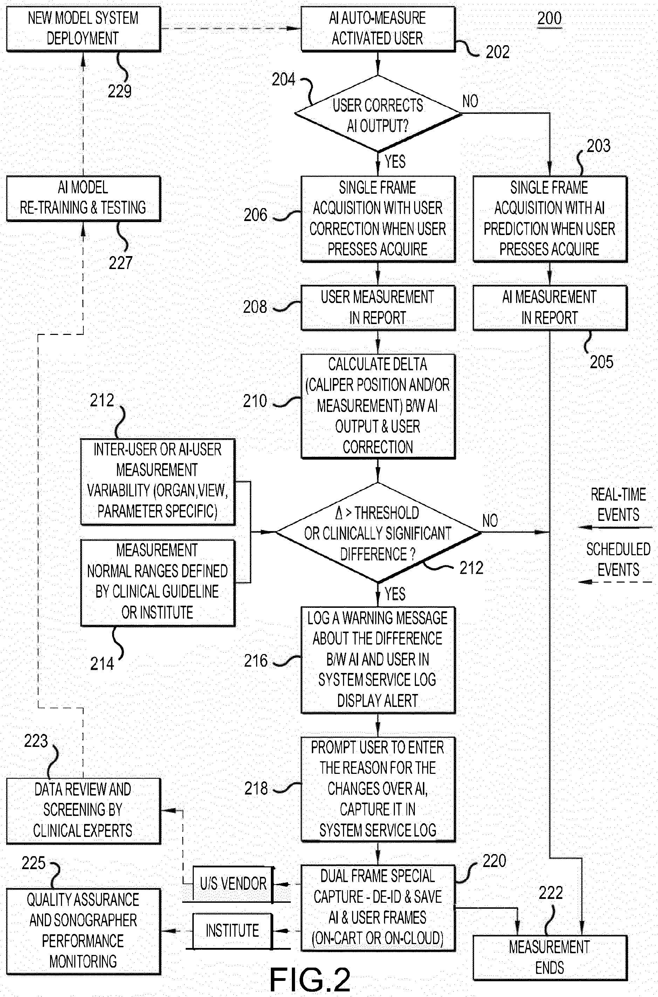

Fig. 2 is a flow chart of a method 200 of executing an AI model to provide a measure of quality assurance of a measurement in accordance with a representative embodiment. The method 200 is contemplated for use in connection with the US system 100, and certain details and aspects of the US system may be common and germane to the method 200. These common aspects and details may not be repeated to avoid obscuring the presently described representative embodiments.

At 202, the AI auto-measurement is activated. As described more fully below, this AI function causes the AI model, based on the US image, to predict the location of calipers in a scanned image and to predict one or more measurements of an organ being examined.

At 204, the user reviews the placement of the calipers and the measurements made by the AI model and determines whether adjustments are required. If the user determines the caliper location and measurements are acceptable relative to certain guidelines described below, the method 200 proceeds to 203 with the acquisition of the image including the AI predicted caliper location and measurement(s). Acquisition may be obtained for example by the user pressing an acquire button on the user interface 122 or the like.

At 205, an AI measurement report is generated and at 222 in this branch of the method, and this aspect of the method is completed. Notably, when the user agrees with the AI output, datasets from measurements without user correction generally is not used for AI model training.

At 204, when the user determines a correction of the measurement, and/or an adjustment of the caliper position is necessary, the method 200 proceeds to 206 at which point the user captures a single frame including the corrections/adjustments made by the user, and the image is archived for later analysis. Capture may occur for example by the user pressing the acquire button or a different button on the user interface 122 or the like.

At 208, user measurement is captured in a report. Notably, the user measurement includes only the corrected/adjusted scan and not the AI prediction after the user makes any corrections. At this point of the method, both the AI output and user correction are captured in system memory for 210. These images are saved and may be accessed by manufacturer of the US system, and/or by quality managers at certain time intervals for evaluation of the user's performance, or to improve the user's skills, or both. Moreover, and as noted below, these images may be used as part of data sets for continual retraining/updating of the AI model training and/or for their quality assurance.

At 210, the algorithm of the AI model compares the adjusted measurements and caliper positions to a threshold to determine whether these measurements and caliper positions are greater than a threshold value. As will be appreciated by one of ordinary skill in the art, certain thresholds can be pre-defined by the US system manufacturer per organ, view, and/or measurement. In other embodiments, the end user of the system may be allowed some degree of customization to the default thresholds.

After the user makes a correction to the caliper position and/or measurement, any differences between the AI predicted position/measurement are determined. Notably, these differences have two components.

The first component comprises a relative difference between AI and user length measurements, and the second component is based on the positional difference between the AI placement of the calipers and the user placement thereof.

At 212 the measurement difference between the AI output and the correction made by this individual user is compared to cutoffs and/or threshold values, which are derived from statistical differences from one user to another, or between the user and the AI measurement using a comparatively large clinical database. At 214, and as described more fully below, the measurements are compared to normal ranges/clinical guidelines.

The second component relates to caliper positioning. Specifically, placing calipers at the correct locations on an organ or other anatomy being scanned must be done accurately. As such, in accordance with a representative embodiment, the difference between positioning of the calipers by the AI and the user are determined. These differences are then compared to cutoffs and/or threshold values defined in the system as described more fully below.

When the differences at 212 are small compared to thresholds or standards, the image is not captured for further use in retraining/updating the AI model or for user evaluation and training. These comparatively small differences between AI model results and user results may be, for example, attributable to variability from one user's measurements/caliper placement to another. In this case, the measurement ends at 222.

When the difference(s) between the AI and user measurements and/or caliper position are clinically significant (i.e., greater than the threshold or cutoff), the method continues to 216 where an alert is provided, and logged. Notably, these data are saved, and as described more fully below, may be used to retrain and update the AI model, and/or for quality assurance and/or user evaluation and training.

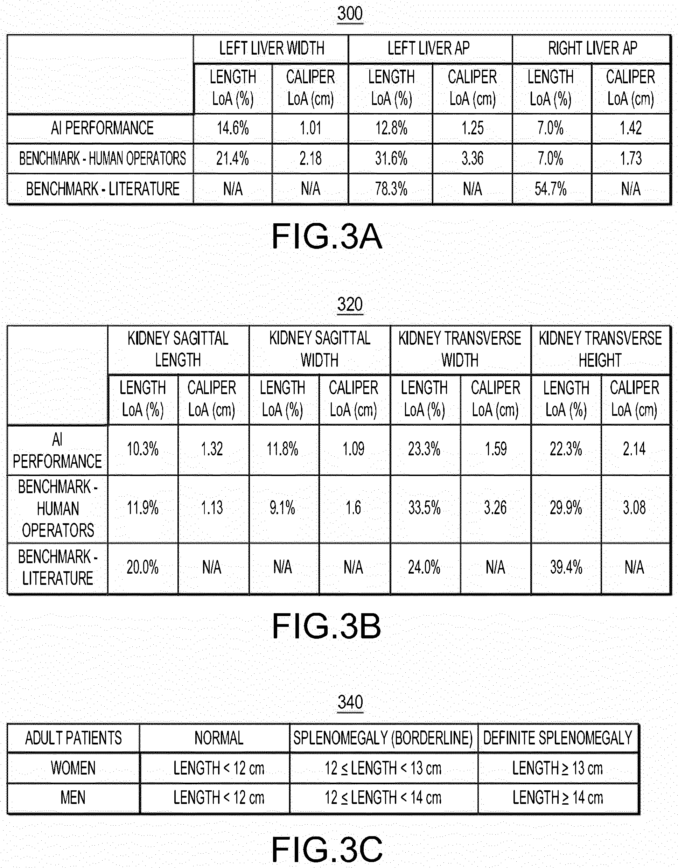

Additionally, at 216 the determination may be made whether the difference between the AI results and the user results spell a clinically significant difference. Just by way of illustration, suppose the AI measurement is within a clinically accepted normal range, but the user measurement is different enough that the user measurement is indicative of a clinically significant difference (e.g., as discussed below in connection with Fig. 3C). Again, these data are saved, and as described more fully below, may be used to retrain/update the AI model, and/or for quality assurance and/or user evaluation and training.

At 218, an optional step in the method includes prompting the user to enter an explanation of the reason for the differences between the AI and user results. These inputs can be used in retraining/updating the AI model, and/or for quality assurance and/or user evaluation and training.

At 220, the method 200 continues with a capture of both the AI results and the user correction to the AI results. These results beneficially have all patient identifying information removed, and are saved at the US system or in the cloud with the measurement ending at 222.

As noted above, and as described more fully below, at 223 the AI results and the user correction to the AI results are provided to the US system manufacturer for AI modeling to provide more ground truth data at 227. These data are then used to retrain/update the AI model, and/or for test data to test the accuracy of the AI model.

Moreover, at 225 the AI results and user correction of the AI results may be used by the institution (e.g., hospital) for quality review, user evaluation, and user training.

At 229, after the retraining or updating of the AI model, as described more fully below, the method comprises deploying the new model (e.g., a newly trained model, or an updated model).

As alluded to above, in accordance with various representative embodiments, inter-user or inter-AI-user measurement variability may be used as a reference threshold to evaluate the difference between the AI prediction (AI measurement or caliper location) and the correction of the AI results by the user. In the tables that follow, measurement variability related to threshold values or clinically significant difference at 212 in Fig. 2 can be captured in different metrics. For purposes of illustration and not limitation, Figs. 3A-3C provided tables Limits of Agreement (LoA) in relative and absolute units for AI measurement and caliper positions. Notably, other measures of differences are contemplated, including for example, standard deviation of the difference between the users for both measurement and caliper position.

Fig. 3A is a table 300 showing reference thresholds for liver size measurement specific to view and measurement parameters. Specifically, Fig. 3A shows left and right lobe liver size (width and thickness) measurement error or variability in longitudinal and transverse view by AI models or human operators. Clinical decision differences are based on the measurement. If the state of the organ changes based on the clinical guideline (from normal to abnormal, or from abnormal to normal) after the user changes the measurement, the system records significant difference.

Fig. 3B shows a table that lists a kidney size (length, width and height) measurement error or variability in longitudinal and transverse view measured by an AI model or human operators. A few useful sources are contemplated for training/retraining and updating the AI model and evaluating the accuracy versus ground truth data from many similar US scans of the predictions made by the AI model. First, the AI model results may be weighed against ground truth data gathered through a comparatively large numbers of measurements/caliper positions. Second, human operator performance can also be relied upon for evaluation and accuracy. Finally, statistics pertaining to kidney size and LoA may be available in the literature. The first two categories are confidential or proprietary information to the US system manufacturer during development and validation (testing) of the AI model. (It is emphasized, the data provided in Figs. 3A and 3B are merely for the purpose of illustration.) Third, some data are derived from literature, but their availability may be limited (as indicated by N/A in Figs. 3A and 3B).

The normal range for the selected measurement defined by clinical guidelines or by the hospital or institute are also useful. Normal ranges may depend on age, gender, body weight and height and other factors. As will be appreciated, abdominal ultrasound is the first-line imaging modality suggested for diagnosis of splenomegaly. Spleen length in the longitudinal view is one important indicator of an enlarged spleen. Fig. 3C provides the spleen length reference range used by an institute for diagnosing normal spleen, splenomegaly (borderline) and definite splenomegaly for adult women and men subjects. With reference to Fig. 3C, normal spleen length is less than approximately 12 cm, with borderline length between 12 cm and 13cm for women, 12 cm and 14 cm for men; whereas an enlarge spleen has a length that is greater than 13 cm for women and 14 cm for men Just by way of example, for a female patient, if the AI measurement were 11 cm, and the user-correction to the AI measurement is greater than 13 cm, a difference that can be deemed a clinically significant difference may be warranted with the user corrected AI measurement indicative of splenomegaly. Referring again to Fig. 2, at 212 the clinically significant difference is detected, and at 216 an alert is given, and the AI-measurement and user correction of the AI measurement are stored, and may be used to train and/or retrain the AI model at 227. Moreover, these stored data and scans may be used by the institution in quality review and/or user evaluation and training.

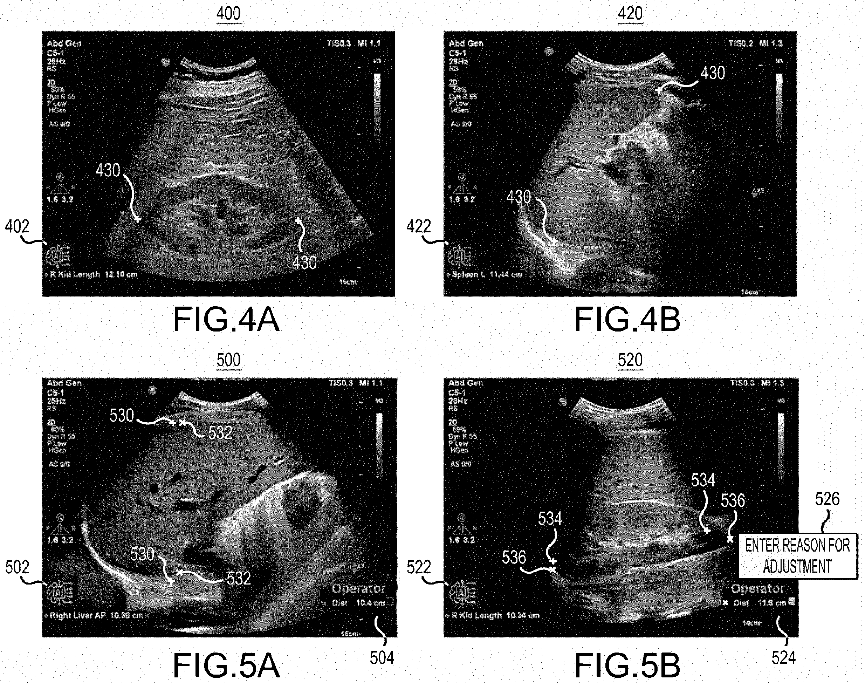

Figs. 4A-5B are useful to show various aspects of the method 200 and its use in retraining and updating the AI model in accordance with a representative. Fig. 4A is a US image overlaid by auto-measure results at 402 from the AI model without any user-correction of a kidney length in longitudinal view. Fig. 4B is a US image showing auto-measure results at 422 by AI without any user-correction of a spleen length in longitudinal view. Notably, markers to annotate AI placed (+) calipers 430 are shown.

By contrast, Fig. 5A is a US image showing user-adjusted caliper positions and new measurement different from AI prediction of a right liver AP thickness measurement with no significant difference between AI prediction and user measurement in accordance with a representative embodiment. Fig. 5B is a US image showing user-adjusted caliper positions and new measurement significantly different from AI prediction of a kidney length measurement in a longitudinal view in accordance with a representative embodiment.

Turning to Figs. 4A and 4B at 400 and 420, respectively, the US system captures the single AI frame after the user proceeds to acquire as part of the regular exam workflow.
Turning to Figs. 5A and 5B at 500 and 520, respectively, two examples are shown in which the user made modifications to the caliper positions predicted by AI model, leading to a different measurement. For the illustration purpose, markers to annotate AI placed (+) calipers 530 and user placed (×) calipers 532 are shown. In Fig. 5A, for right liver anteroposterior (AP) measurement, the posterior caliper by the AI model is slightly deeper away from liver parenchyma and diaphragm. As such, the user moved the marker up. The user correction changed the liver measurement from 10.98 cm to 10.4 cm. In this embodiment, both of an AI model measurement 502 and a user ("Operator") measurement 504, are displayed such that the measurement difference is more apparent. In Fig. 5B, in the right kidney length measurement, there are moderate acoustic shadows on both sides of the image which may impact the AI predication of the caliper position. In Fig. 5B, the user expanded the caliper range further out to cover the kidney lower pole and upper pole, and the user correction changed the kidney measurement from 10.34 cm to 11.8 cm. In this representative embodiment, both of an AI model measurement 522 and a user ("Operator") measurement 524 are displayed such that the measurement difference is more apparent. For the illustration purpose, markers to annotate AI placed (+) calipers 534 and user placed (×) calipers 536 are shown.

By methods described more fully below, the US system detects a significant difference between AI caliper position and the user correction of the caliper position. This results in the dual-frame special capture described above at 220 in the method 200 of Fig. 2. In this case, the US system executes dual acquire, and captures both frames. As noted above, these data may be used to retrain and/or update the AI model. Concurrent to this dual-frame special capture (both images from Fig. 5B), a warning message with timestamps, is written in the Ultrasound System Service or Workflow Log file to record the significant difference between the paired frames. The text message could contain the AI measurement with respective caliper positions, user measurement with respective caliper positions and the measurement difference. These data can be used by the US system manufacturer to understand AI performance (e.g. statistics of acceptance rate) and direct a reviewer's attention to review those measurements for model improvement. As such, this dual-frame special capture at 220 of Fig. 2 can be provided via 223 of the method 200 of Fig. 2, not only to evaluate the performance of the AI model, but also to retrain or update the AI model. Finally, with reference again to 218 of the method 200 of Fig. 2, a prompt 526 to capture operator's reason(s) for such a user correction via text or voice may be presented. The resulting user input can be embedded in the Service/Workflow log too, to further assist image/measurement interpretation. If user prefers not to provide any input during the exam, this option can be turned off in System Setup. As alluded to above, an algorithm to provide the measures of the degrees of difference between the AI model measurement and caliper location and the user-corrected AI measurements and caliper locations is disclosed. The algorithm has two parts. When or more of the below two situations occurs, the US system records significant difference between AI results and the user correction. As noted above, these significant differences can result from exceeding a threshold or a clinically significant difference, or both.

The first part comprises a measurement numerical value difference. Here we suggest calculating two metrics: length measurement difference *δ_{L}* Eq. (1) and the maximum caliper position difference *δ_{caliper}* Eq. (3). If one of these two metrics is greater than its respective reference threshold, the system records significant difference: ${δ}_{L} = \left|\frac{{L}_{AI} - {L}_{user}}{{L}_{user}}\right| ∗ 100 %$ (1) ${δ}_{{caliper}_{i}} = \sqrt{{\left({x}_{{AI}_{i}}-{x}_{{user}_{i}}\right)}^{2} + {\left({y}_{{AI}_{i}}-{y}_{{user}_{i}}\right)}^{2}}$ (2) ${δ}_{caliper} = \left.{\left〚MAX\left(δ\right.\right〛}_{{caliper}_{i}}\right) , i = 1 , 2 for two calipers$ (3) 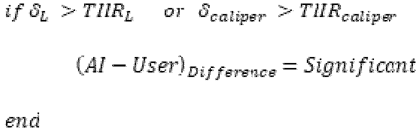 where L denotes a length measurement (e.g., organ length, width, height, thickness, diameter, or other dimensional measure); *L_{AI}* stands for measurements by the trained AI model; and *Lᵤₛₑᵣ* stands for measurement the measurement after user adjustment or editing; *δ* annotates the difference in length measurement or caliper position. When *δ* is associated with *L*, we calculate the relative difference using *Lᵤₛₑᵣ* for the denominator.

When *δ* is associated with caliper position difference, the absolute difference is calculated by measuring the distance between the AI predicted caliper and user adjusted caliper. Because any 1-D measurement is defined by a pair of calipers and each caliper has a coordinate (xi,yi) on the image, Equation (2)

Next, a maximum value of *δ_{caliper}* = *MAX*( *δ*_{*caliper*₁}), *i* = 1, 2 for two capliers is taken to represent the final caliper position difference for this 1-D measure, where the two thresholds are *THR_{L}* and *THR_{caliper}*

The second part comprises a clinical decision difference based on the measurement. If the state of the organ changes based on the clinical guideline (from normal to abnormal, or from abnormal to normal) after the user changes the measurement, the system records significant difference: 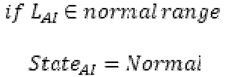 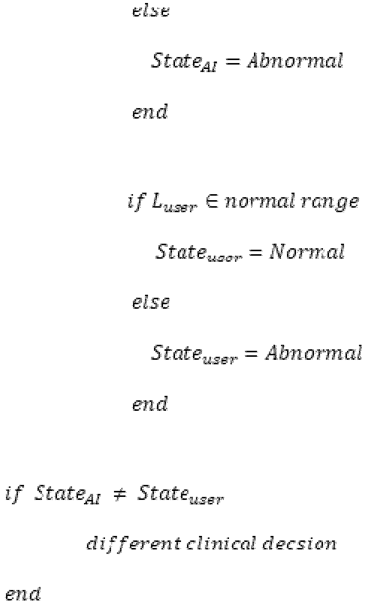 where *State_{AI}* denotes the state of the organ based on AI model measurements and *Stateᵤₛₑᵣ* denotes the state of the organ based user corrected measurements.

Just by way of illustration, referring to Fig. 5A, the AP length measurement difference for the liver right side is ${δ}_{L} = \frac{10.98 - 10.4}{10.4} = 5.6 %$, which smaller than the threshold 7.0% in Table 300. And the caliper displacement is 0.70 *cm* and 0.82 *cm*, which are both smaller than the threshold of 1.42 *cm*. Therefore, the difference is deemed insignificant. As a result, no special capture of dual frames will be executed. By contrast, as shown in Fig. 5B, the measurement difference of the kidney length is ${δ}_{L} = \frac{11.8 - 10.34}{11.8} = 12.4 %$, greater than the reference threshold 10.3% shown in Table 320. Moreover, the lower pole caliper position displacement *δ_{caliper}* = 1.50 *cm*, greater than the reference threshold 1.32 *cm*. The system determines the difference to be significant. The thresholds referenced here are 10.3% and 1.3.

As will be appreciated, based on these algorithms, data for retraining and/or updating the AI model (discussed below) are gathered, as are measure of the performance of the model.

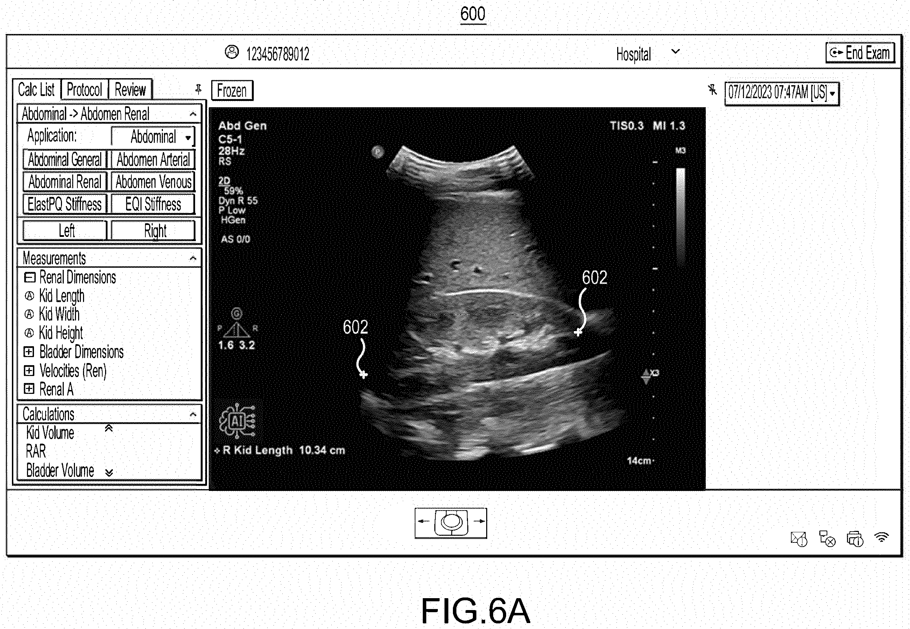

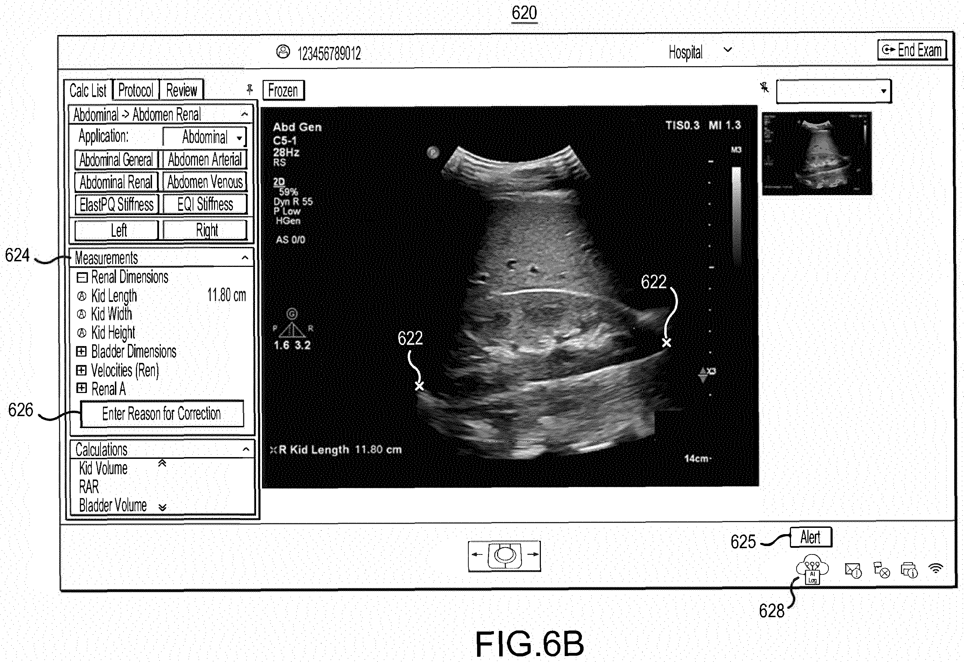

Figs. 6A and 6B show the two steps in the acquisition workflow for the case of Fig. 5B with the display on the system monitor (images 600 and 620, respectively) and data described above, and thus how the length measurement by the AI model (Fig. 6A and illustrated by calipers 602 (+)) and user-corrected length measurement (Fig. 6B and illustrated by calipers 622 (X)) are archived and documented for a scan of a kidney in accordance with a representative embodiment. Notably, the length measurement made by the AI model (10.34 cm) in Fig. 6A is corrected by adjusting the positions of the calipers by the user in Fig. 6B. The correction leads to a new measurement of 11.80 cm as shown in Fig.6B as illustrated by user-adjusted calipers 622. After the user presses the button "acquire" the images (e.g., at 204 in Fig. 2), the user-corrected frame on the main display is captured as part of the exam protocol in the foreground, as indicated in the thumbnail area on the right side of the main display in Fig. 6B. Concurrently, the new measurement is displayed in the measurement panel on the left side of the main display at measurement text block 624. The measurement panel may further include a prompt 626 for the user to enter the reason for adjusting the measurement. This means the new measurement by the user is accepted as the final value entering the measurement report. In the background, the US system detects the difference between AI and user correction to be greater than the thresholds, so an alert message is automatically recorded in the System Service/Workflow Log file, to alert the clinical reviewer for quality control and the US system manufacturers for quality review and user training, AI retraining/updating and improvement with these new data, respectively. Notably, an alert message 625 may be displayed when the user wants to enter the reasons for the correction.

Again, referring to the method 200 of Fig. 2, these actions occur at 220, 223 and 225, described above. Simultaneously, the US system performs the dual capture to archive the paired frames of the AI model measurement and user-correction of the AI model length measurements (images 600, 620) in a designated storage memory either on-cart or on-cloud. In the foreground, a new icon 628 with annotation "AI Log" is added to the main display (lower right corner) to signal that there is AI data for review in the logs. Notably, in another representative embodiment showing the dual capture of images, a single frame is captured and includes user and user-correction of the AI model length measurements and AI model measurement both annotated and displayed on the same image frame. This annotation could be done using distinct colors, or gray level variation to differentiate the model calipers predicted by the AI model, and corrections to these prediction by the user-placed calipers and measurements, either by highlighting user results or lowlighting the AI model predictions.

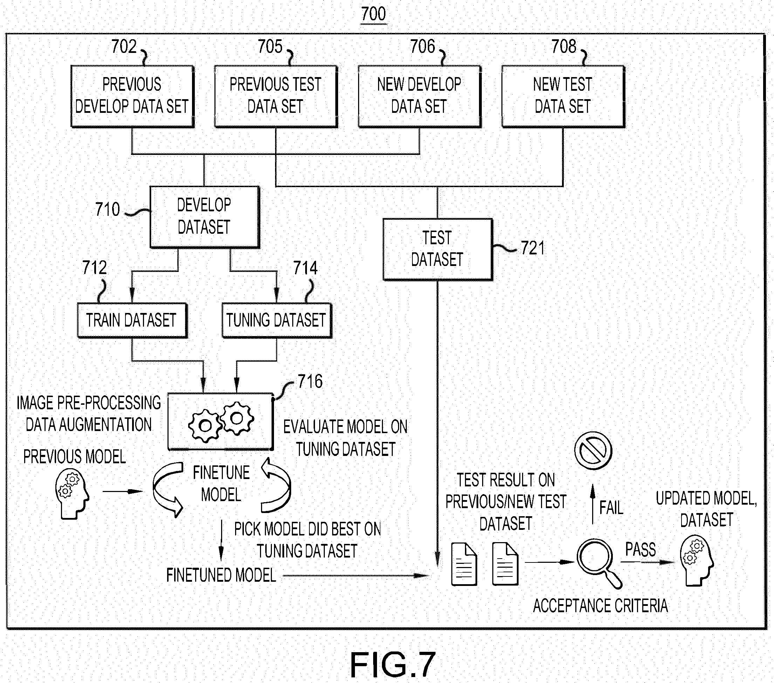

Fig. 7 is a flow chart of a method 700 of updating and retraining an AI computational model in accordance with a representative embodiment. Various aspects and details of the updating and retraining are common to those described above in connection with the representative embodiments of Figs. 1-6B, and may not be repeated in order to avoid obscuring the presently described representative embodiments.

Referring to Fig. 7, a development data set 702 comprises ground truth data from scans include images, measurements with caliper locations made by users (e.g., sonographer, radiologists and other clinicians). As will be appreciated by one of ordinary skill in the art, the previous development data set 702 is used in training of the model, and a previous test data set 704, used for performance evaluation is also stored and used during model training and updating.

A new development data set 706 is garnered from a selected number of scans, and comprises user corrected measurements and caliper locations collected in recent scans using AI enabled auto-measure tool. Just by way of illustration, the new development data set 706 may comprise data from 100 scans, whereas the development data set 702 may comprise data from thousands or more of scans/measurement. Finally, a new test data set 708 is stored for evaluation of the model, and is combined with the previous test data set 705 to provide the updated test data set 721. As shown, the updated test data set 721 is used to test an updated AI model, by a known method illustratively a known error calculation. The results of the test are either pass or fail based on acceptance criteria as shown in Fig. 7.

The development data set 710 is separated to a training data set 712 and a tuning data set 714, and are provided separately to the current AI model using image pre-processing and augmentation 716 as shown. The current AI model is run using these newer data sets and evaluated for performance. This updated model with updated parameters is then optimized using the tuning data set 714 for performance evaluation. As needed, the updated AI model may then be fine-tuned by adjusting hyperparameters and the updated model is again run for performance evaluation. This process may be repeated to determine the most accurate AI model based on the tuning data set 714. The best retrained (fine-tuned) AI model is then evaluated as shown, and may be provided for use in the US systems of various representative embodiments as the updated AI model.

Accordingly, and as alluded to above, in accordance with various representative embodiments described above, data captured at 220 of Fig. 2, is provided for AI model retraining and testing to continually update AI model being deployed in the US system. As will be appreciated, and as alluded to above, this continual retraining and testing of the AI model provides a continual improvement to the function of the US system, and as a result provides more reliable AI model measurements and caliper locations.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A computer-implemented method of using a trained artificial intelligence, AI, model, the method comprising:
applying the trained AI model to an ultrasound image to acquire automatically a trained AI model measurement of an anatomy, wherein the automatically acquired trained AI model measurement of the anatomy provides a pair of calipers positioned on the ultrasound image to obtain a length or distance measurement;
detecting when a user corrects the trained AI model measurement by adjusting a caliper position to obtain a correction to the trained AI model measurement;
determining a measurement difference between the trained AI model measurement and the correction to the trained AI model measurement;
determining when the measurement difference between the trained AI model measurement and the correction to the trained AI model measurement is greater than a threshold;
when the measurement difference between the trained AI model measurement and the correction is greater than the threshold, or leads to a different clinical decision, providing an alert indicative of the measurement difference between the trained AI model measurement and the correction, or providing an alert indicative of the different clinical decision;
archiving paired images comprising the trained AI model measurement and the correction to the trained AI model measurement; and
determining an improvement to the trained AI model based on the measurement difference between the trained AI model measurement and the correction to the trained AI model measurement.

2. The method of claim 1, further comprising determining when the trained AI model measurement and the correction to the trained AI model measurement lead to a clinically significant measurement difference, by evaluating the measurement with reference to a normal range.

3. The method of claim 1, wherein normal ranges are provided for a selected measurement defined by clinical guidelines or by an institutional guideline, and wherein the method further comprises comparing the normal ranges to the trained AI model measurement, and/or the correction to the trained AI model measurement.

4. The method of any of claims 1 or 3, wherein the measurement difference comprises a difference between the length or distance measurement and a maximum caliper position difference.

5. The method of any of claims 1 to 4, further comprising providing a set of thresholds related to measurement variability.

6. The method of claim 5, wherein thresholds in the set of thresholds are pre-defined specifically for an individual organ, an image view, anatomical measurement parameters, and caliper positions.

7. The method of any of claims 4 to 6, further comprising determining, with the trained AI model, a performance evaluation of a user based on the difference between the length or distance measurement and the maximum caliper position difference.

8. The method of claim 7, wherein determining the performance evaluation of the user is further based on the difference between the caliper position and the correction to the position.

9. The method of any of claims 1 to 8, further comprising:
retraining the trained AI model with archived data from a plurality of measurements.

10. The method of claim 4, further comprising determining a difference between the caliper position and a correction to the position;
determining when the caliper position and the correction is greater than a threshold; and
when the difference is greater than the threshold providing an alert indicative of the difference exceeding the threshold.

11. The method of claim 10, further comprising determining a measure of quality assurance based on the difference between the caliper position and the correction to the position.

12. The method of any of claims 1 to 11, further comprising:
providing a user prompt to enter reasons for the correction;
receiving the reasons; and
archiving the reasons with the archived paired images.

13. The method of claim 12, further comprising transmitting to a remote system the ultrasound image with the trained AI model measurement of the anatomy, the correction to the trained AI model measurement and the receive reason, to perform re-training of the trained AI model, or output a measurement quality report at the remote system, or both.

14. A tangible, non-transitory computer readable medium that stores instructions, which when executed by a processor, cause the processor to perform the method of any of claims 1 to 13.

15. An ultrasound system comprising a trained artificial intelligence, AI, model, the ultrasound system comprising:
a processor;
a tangible, non-transitory computer readable medium that stores instructions, which when executed by the processor, cause the processor to:
apply the trained AI model to an ultrasound image to acquire automatically a trained AI model measurement of an anatomy, wherein the automatically acquired trained AI model measurement of the anatomy provides a pair of calipers positioned on the ultrasound image to obtain a length or distance measurement;
detect when a user corrects the trained AI model measurement by adjusting a caliper position to obtain a correction to the trained AI model measurement;
determine a measurement difference between the trained AI model measurement and the correction to the trained AI model measurement;
determine when the measurement difference between the trained AI model measurement and the correction to the trained AI model measurement is greater than a threshold;
when the measurement difference between the trained AI model measurement and the correction is greater than the threshold, or leads to a different clinical decision, provide an alert indicative of the measurement difference between the trained AI model measurement and the correction, or provide an alert indicative of the different clinical decision;
archive paired images comprising the trained AI model measurement and the correction to the trained AI model measurement; and
determine an improvement to the trained AI model based on the measurement difference between the trained AI model measurement and the correction to the trained AI model measurement.
